# EUROPÄISCHE PATENTSCHRIFT

(11) **EP 0 334 164 B1**
(45) Veröffentlichungstag und Bekanntmachung des Hinweises auf die Patenterteilung: **27.10.1993**
(21) Anmeldenummer: 89104493.5
(22) Anmeldetag: 14.03.1989
(51) Int. Cl.: A61K 31/55

(54) **Antihypertensives Kombinationspräparat**
Antihypertensive combination preparation
Préparation combinée contre l'hypertension

(30) Priorität: 24.03.1988 CH 1114/88
(43) Veröffentlichungstag der Anmeldung: 27.09.1989
(73) Patentinhaber: F. HOFFMANN-LA ROCHE AG, 4002 Basel (CH)
(72) Erfinder: Kleinbloesem, Cornelis, Dr., CH-4103 Bottmingen (CH)
(74) Vertreter: Lederer, Franz, Dr.

(56) Entgegenhaltungen:
- EP-A- 0 211 220
- EP-A- 0 224 810
- Dialog 6785862, file 155 8908762; G.G. Belz et al. Band 25, Nr. 5, 05.11.88 Seiten 547-556.

## Beschreibung

Die vorliegende Erfindung betrifft ein pharmazeutisches Kombinationspräparat, das zur Behandlung von Hypertonie geeignet ist, enthaltend bestimmte Alkylaminoalkoxy-phenyl-, -naphthyl- oder -indolyl-Derivate sowie bestimmte Pyridazodiazepine.

Die erwähnten Phenyl-, Naphthyl- und Indolyl-Derivate sind bekannte β-Blocker. Sie zeigen unter anderem eine ausgeprägte antihypertensive Wirkung und können bei der Behandlung von Hypertonie aller Schweregrade verwendet werden.

In ähnlicher Weise handelt es sich bei den erwähnten Pyridazodiazepinen um bekannte ACE-Hemmer, die sich ebenfalls für die Behandlung von Hypertonie eignen; sie weisen eine blutdrucksenkende Wirkung auf, ohne die Herzfrequenz zu erhöhen (vgl. Deutsche Offenlegungschrift 3317290).

Die gleichzeitige Applikation eines β-Blockers und eines ACE-Hemmers ist in der Fachliteratur bereits beschrieben worden. Amery et al. berichten in Clinical Science, 61, 441s-444s (1981), dass bei Patienten, deren Bluthochdruck mit Captopril behandelt wird, mittels zusätzlicher Verabreichung von Propranolol eine weitere Blutdrucksenkung erzielt werden konnte. Dieser Befund konnte jedoch später nicht mehr bestätigt werden. So berichten beispielsweise MacGregor et al. in Journal of Cardiovascular Pharmacology, 7, S82-S87 (1985), dass bei ihren Untersuchungen die zusätzliche Applikation von Propranolol bei über einen längeren Zeitraum mit Captopril behandelten Patienten keine zusätzliche Blutdrucksenkung bewirkte. Als mögliche Erklärung für das Ausbleiben einer zusätzlichen Blutdrucksenkung geben die Autoren an, dass wahrscheinlich β-Blocker und ACE-Hemmer - mindestens zum Teil - den gleichen Wirkungsmechanismus besitzen, nämlich Hemmung des Renin-Angiotensin-Systems, weshalb bei einer Kombinationstherapie auch nicht einmal ein additiver Effekt erwartet werden kann. Diese Meinung hat sich seither in der Fachwelt durchgesetzt [vgl. Eur. J. Clin. Pharmac., 32, 229-235 (1987)], weshalb auch heute noch bei Hypertonie eine kombinierte Therapie von β-Blockern und ACE-Hemmern nicht empfohlen werden kann [vgl. Journal of Cardiovascular Pharmacology, 9, Suppl. 3, 2-5 (1987)].

Es besteht das Bedürfnis, eine pharmazeutische Kombination bereitzustellen, auf die möglichst alle Patienten ansprechen, deren Verabreichung zu einer Senkung des Blutdruckes führt, ohne gleichzeitig die Herzfrequenz zu erhöhen, und in welcher die Dosen der Einzelkomponenten reduziert und unerwünschte Nebenwirkungen, die jeweils bei der notwendigen Dosierung in der Monotherapie auftreten, unterdrückt werden können.

Im Rahmen der vorliegenden Erfindung konnte festgestellt werden, dass bei Verabreichung der erfindungsgemässen Kombination von einem β-Blocker mit einem ACE-Hemmer signifikant mehr Patienten auf die Behandlung ansprechen als bei den Monotherapien und die blutdrucksenkenden Eigenschaften der beiden Einzelkomponenten nicht nur addiert, sondern teilweise sogar potenziert werden, wodurch die wirksamen Dosen der beiden Einzelkomponenten herabgesetzt werden können. Darüberhinaus war nicht vorauszusehen, dass gleichzeitig auch noch die Wirkungsdauer erheblich verlängert wird.

Die erfindungsgemässe antihypertensive Kombination besitzt demnach folgende Vorteile:
1. Der Anteil an Patienten, die auf die Behandlung ansprechen, wird signifikant erhöht;
2. die zu applizierenden Wirkstoffmengen werden reduziert;
3. unerwünschte Nebenwirkungen werden eliminiert bzw. stark reduziert;
4. die Herzfrequenz wird nicht beeinflusst;
5. die Wirkungsdauer wird verlängert und
6. ein gleichmässiger Wirkungsverlauf wird erreicht.

Die Erfindung betrifft somit ein neues pharmazeutisches Kombinationspräparat zur Behandlung von Hypertonie, enthaltend einen β-Blocker der allgemeinen Formel 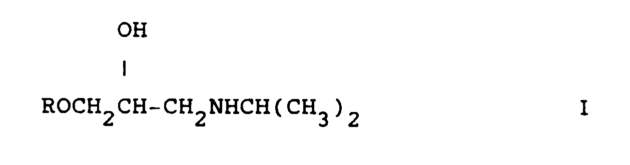
worin R Phenyl, welches gegebenenfalls durch nieder-Alkenyloxy, nieder-Alkoxy-nieder-alkyl, Aminocarbonyl-nieder-alkyl oder nieder-Alkoxy-nieder-alkoxy-nieder-alkyl substituiert ist, oder Naphthyl, Indolyl oder Dihydroxytetrahydronaphthyl bedeutet,
und ein Pyridazodiazepin der allgemeinen Formel 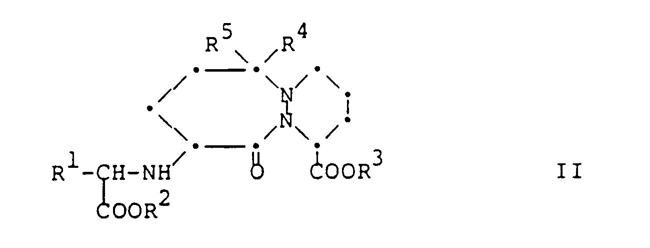
worin R¹ Aryl-nieder-alkyl, R² und R³ je Wasserstoff oder nieder-Alkyl und R⁴ und R⁵ je Wasserstoff oder zusammen eine Oxogruppe bedeuten,
wobei die Wirkstoffe entweder in Form ihrer freien Basen, ihrer Hydrate oder ihrer pharmazeutisch verwendbaren Salze vorliegen.

Der in dieser Beschreibung verwendete Ausdruck "nieder" bezieht sich auf Reste, welche 1-4 Kohlenstoffatome aufweisen.

Der Arylrest in Aryl-nieder-alkyl ist eine Phenylgruppe, welche durch Halogen (d.h. Fluor, Chlor, Brom oder Jod), nieder-Alkyl, nieder-Alkoxy, Trifluormethyl, Phenyl und dergleichen ein- oder mehrfach substituiert sein kann. Beispiele von Aryl-nieder-alkylgruppen sind Benzyl, 4-Chlorbenzyl, 2-Phenyläthyl, 3-Phenylpropyl, 3-(4-Chlorphenyl)propyl, 3-(4-Methoxyphenyl)propyl, 4-Phenylbutyl und dergleichen.

Das Gewichtsverhältnis von β-Blocker zu Pyridazodiazepin beträgt zweckmässigerweise etwa 0,5:1 bis 500:1, vorzugsweise 1:1 bis 50:1, ganz besonders bevorzugt 1:1 bis 5:1, in Bezug auf freie Base(n), wobei das Gewichtsverhältnis vom verwendeten β-Blocker und Pyridazodiazepin abhängt.

Vorteilhafterweise beträgt die mittels der Kombination pro Tag zu verabreichende Dosis 5-320 mg eines β-Blockers und 1-5 mg eines Pyridazodiazepins. Im allgemeinen beträgt die täglich zu verabreichende Gesamtmenge von einem β-Blocker und einem Pyridazodiazepin maximal 325 mg. Wird ein Hydrat oder in pharmazeutisch verwendbares Salz eingesetzt, so sind die obigen Werte entsprechend zu ändern.

Gegenstand der vorliegenden Erfindung sind somit
- eine pharmazeutische Zubereitung, enthaltend einen β-Blocker der Formel I und ein Pyridazodiazepin der Formel II;
- die Herstellung einer pharmazeutischen Zubereitung, welche dadurch gekennzeichnet ist, dass man ein Gemisch von einem β-Blocker der Formel I und einem Pyridazodiazepin der Formel II in eine galenische Darreichungsform bringt;
- die Verwendung einer Kombination von einem β-Blocker der Formel I und einem Pyridazodiazepin der Formel II bzw. einer pharmazeutischen Zubereitung, enthaltend einen β-Blocker der Formel I und ein Pyridazodiazepin der Formel II, zur Bekämpfung bzw. Verhütung von Krankheiten, insbesondere von Kreislauferkrankungen, ganz besonders bei der Bekämpfung bzw. Verhütung von Hypertonie und deren Folgeerkrankungen, wie z.B. die Herzinsuffizienz, ohne Erhöhung der Herzfrequenz.

Besonders geeignete β-Blocker sind solche der Formel I, worin R α-Naphthyl, Indol-4-yl, 2-Allyloxyphenyl, 4-Methoxyäthylphenyl, 4-Aminocarbonylmethyl, 4-Isopropyloxyäthyloxymethylphenyl oder 6,7-Dihydroxy-5,6,7,8-tetrahydronaphth-1-yl bedeutet. Ganz besonders geeignet sind solche β-Blocker der Formel I, worin R α-Naphthyl, Indol-4-yl, 2-Allyloxyphenyl, 4-Aminocarbonylmethyl oder 4-Isopropyloxyäthyloxymethylphenyl bedeutet.

Die geeignetsten Vertreter aus der Gruppe der β-Blocker der Formel I sind Propranolol und Bisoprolol.

Besonders geeignete Pyridazodiazepine sind solche der Formel II, worin R¹ Aryl-nieder-alkyl, R² nieder-Alkyl, R³ Wasserstoff und R⁴ und R⁵ Wasserstoff oder zusammen eine Oxogruppe bedeuten.

Ganz besonders geeignete Pyridazodiazepine sind solche der Formel II, worin R¹ Phenyl-nieder-alkyl, R² nieder Alkyl und R³, R⁴ und R⁵ Wasserstoff bedeuten.

Der geeignetste Vertreter aus der Gruppe der Pyridazodiazepine der Formel II ist die 9(S)-[1-(S)-Aethoxycarbonyl-3-phenylpropylamino]octahydro -10-oxo-6H-pyridazo[1,2-a]-[1,2]diazepin-1(S)-carbonsäure (nachstehend Cilazapril genannt).

Die bevorzugteste erfindungsgemässe Kombination ist eine Kombination von Cilazapril mit Bisoprolol aufgrund der folgenden, die beiden Wirkstoffe kennzeichnenden Eigenschaften:
- Aehnliches pharmakokinetisches Profil, d.h. eine hohe Bioverfügbarkeit und lange Eliminationshalbwertszeit;
- lange Wirkungsdauer (d.h. nur eine Applikation täglich);
- hohe Spezifizität der beiden Wirkstoffe für ihren jeweiligen pharmakologischen Angriffspunkt und
- hohe Wirksamkeit der beiden Wirkstoffe sowie ungefähre Aequipotenz auf Basis der oralen Dosierung der freien Basen.

Aus diesen Gründen eignen sich die beiden Wirkstoffe Cilazapril und Bisoprolol besonders gut für eine therapeutische Verwendung in Form einer fixen Kombination.

Zweckmässigerweise liegen Propranolol und Bisoprolol als pharmazeutisch verwendbare Salze vor, während Cilazapril als pharmazeutisch verwendbares Salz oder Hydrat vorliegt. In der Regel ist Propranolol als Hydrochlorid bzw. Bisoprolol als Fumarat und Cilazapril in Form des entsprechenden Monohydrates bzw. Hydrobromides in der Kombination enthalten. Vorzugsweise beträgt das Gewichtsverhältnis von β-Blocker zu Pyridazodiazepin bei der Kombination von Propranolol mit Cilazapril etwa 20:1 bis 50:1 und bei der Kombination von Bisoprolol mit Cilazapril etwa 1:1 bis 5:1, bezogen auf die freien Basen.

Mit der erfindungsgemässen Kombination kann mit geringen Wirkstoffdosen eine regelmässige und lang andauernde Blutdrucksenkung ohne Erhöhung der Herzfrequenz bei gleichzeitiger guter Verträglichkeit und geringer Toxizität erzielt werden.

Die vorteilhafte, zumindest additive blutdrucksenkende Wirkung der erfindungsgemässen Kombination gegenüber derjenigen der beiden Einzelkomponenten sowie das gute Ansprechen der Patienten auf diese konnten in den beiden nachfolgend beschriebenen humanexperimentellen Versuchen mit einer Kombination von Cilazapril mit Propranolol gezeigt werden, welche in Uebereinstimmung mit der Deklaration von Helsinki, in der Fassung von Venedig, durchgeführt wurden.
A) Sechs gesunde männliche Probanden, die vorgängig des Versuchs ihr schriftliches Einverständnis dazu gegeben hatten, nahmen an dieser 4-Weg-Crossover-Studie teil, die nach dem Doppelblindverfahren durchgeführt wurde und folgende 4 Behandlungsabschnitte von jeweils 7 Tagen umfasste, wobei zwischen den einzelnen Behandlungsabschnitten jeweils eine Pause von > 7 Tagen eingeschaltet wurde:
   (1) Placebo plus Placebo
   (2) 2,5 mg Cilazapril plus Placebo
   (3) 120 mg Propranolol plus Placebo
   (4) 2,5 mg Cilazapril plus 120 mg Propranolol.

Damit kein Proband ein gesundheitliches Risiko eingehen musste, wurde vor Beginn der Studie von jedem Probanden die Anamnese erhoben, und man unterzog ihn einer Allgemeinuntersuchung, die mit folgenden Kontrollen verbunden war: EKG (12 Ableitungen) sowie Laboruntersuchungen der blutbildenden, hepatischen und renalen Funktionen. Die wichtigsten Laborparameter wurden auch jeweils zu Beginn und am Ende der Pausen zwischen den einzelnen Behandlungsabschnitten überprüft und ergaben keinerlei Pathologika.

Die Probanden hatten jeweils an den Versuchstagen 0 bis 6 nüchtern zu erscheinen, d.h. sie hatten seit dem vorigen Abend weder gegessen, getrunken noch geraucht. Nach 5-minütiger Ruhepause im Sitzen wurden systolischer und diastolischer Blutdruck und Puls gemessen. Anschliessend konnten die Probanden frühstücken, und man verabreichte ihnen unter Ueberwachung die Medikation mit 100 ml Leitungswasser und entliess sie nach Hause. Am Versuchstag 7 erschienen die Probanden ebenfalls nüchtern; die Untersuchungen wurden jedoch im Liegen bei 15° erhobenem Oberkörper durchgeführt. Nach jeweils 15-minütiger Ruhepause im Liegen wurden Blutdruck und Puls zu den Zeitpunkten -2, 1, 2, 6, 8 und 24 Stunden gemessen.

Die während der 7-tägigen Periode beim Plasmakonzentrationsminimum der Präparate erfolgten Messungen liessen keine wesentlichen Unterschiede zwischen den verschiedenen Medikationen erkennen, während am 7. Tag deutlich sichtbar unterschiedliche Wirkungen beobachtet werden konnten.

Unter der Monotherapie mit Propranolol kam es zu einem Abfall des systolischen und diastolischen Blutdrucks um ca. 7 mmHg und einer Abnahme der Herzfrequenz um ca. 8 Schläge/Minute. Unter der Monotherapie mit Cilazapril sanken systolischer und diastolischer Druck fast wie unter der Monotherapie mit Propranolol (ca. 7 mmHg), während die Herzfrequenz um ca. 5 Schläge/Minute zunahm. Unter der Kombinationstherapie mit Propranolol und Cilazapril sank der Blutdruck stärker als unter der Einzelgabe beider Komponenten, und die mittlere maximale Blutdrucksenkung betrug ca. 15 mmHg. Am Behandlungstag 7 ist ausserdem die starke Blutdrucksenkung über mehr als 8 Stunden nach Tabletteneinnahme beobachtbar. Die Herzfrequenz wird unter der Kombinationstherapie weder gesenkt noch erhöht.

Die im oben beschriebenen humanexperimentellen Versuch erhaltenen Resultate bezüglich Blutdruck sind in den nachstehenden Tabellen zusammengefasst.

### Systolische Blutdruckwerte in mmHg am 7. Versuchstag

| (1) Placebo plus placebo | | | | | | |
|---|---|---|---|---|---|---|
| Proband/Std | -2 | 1 | 2 | 6 | 8 | 24 |
| 1 | 107 | 113 | 102 | 107 | 111 | 120 |
| 2 | 113 | 109 | 112 | 112 | 112 | 122 |
| 3 | 106 | 107 | 106 | 105 | 108 | 104 |
| 4 | 115 | 111 | 114 | 121 | 119 | 121 |
| 5 | 120 | 112 | 118 | 136 | 124 | 142 |
| 6 | 118 | 111 | 120 | 122 | 124 | 100 |
| Mittelwert | 1̅1̅3̅ | 1̅1̅1̅ | 1̅1̅2̅ | 1̅1̅7̅ | 1̅1̅6̅ | 1̅1̅8̅ |
| SEM * | 2 | 1 | 3 | 5 | 3 | 6 |

| | | | | | | |
|---|---|---|---|---|---|---|
| * SEM = Standard Error Mean = mittlere Standardabweichung | | | | | | |

| (2) 2,5 mg Cilazapril plus Placebo | | | | | | |
|---|---|---|---|---|---|---|
| Proband/Std | -2 | 1 | 2 | 6 | 8 | 24 |
| 1 | 96 | 102 | 84 | 92 | 88 | 108 |
| 2 | 115 | 113 | 111 | 110 | 112 | 134 |
| 3 | 105 | 100 | 97 | 102 | 100 | 106 |
| 4 | 110 | 108 | 106 | 116 | 124 | 120 |
| 5 | 112 | 118 | 108 | 116 | 114 | 112 |
| 6 | 117 | 111 | 115 | 106 | 108 | 126 |
| Mittelwert | 1̅0̅9̅ | 1̅0̅9̅ | 1̅0̅4̅ | 1̅0̅7̅ | 1̅0̅8̅ | 1̅1̅8̅ |
| SEM * | 3 | 3 | 5 | 4 | 5 | 4 |

| | | | | | | |
|---|---|---|---|---|---|---|
| * SEM = Standard Error Mean = mittlere Standardabweichung | | | | | | |

| (3) 120 mg Propranolol plus Placebo | | | | | | |
|---|---|---|---|---|---|---|
| Proband/Std | -2 | 1 | 2 | 6 | 8 | 24 |
| 1 | 107 | 104 | 103 | 104 | 102 | 116 |
| 2 | 119 | 108 | 113 | 114 | 112 | 128 |
| 3 | 101 | 110 | 99 | 96 | 103 | 112 |
| 4 | 113 | 104 | 106 | 112 | 114 | 108 |
| 5 | 120 | 111 | 108 | 122 | 112 | 112 |
| 6 | 103 | 100 | 108 | 112 | 104 | 112 |
| Mittelwert | 1̅1̅1̅ | 1̅0̅6̅ | 1̅0̅6̅ | 1̅1̅0̅ | 1̅0̅8̅ | 1̅1̅5̅ |
| SEM * | 3 | 2 | 2 | 4 | 2 | 3 |

| | | | | | | |
|---|---|---|---|---|---|---|
| * SEM = Standard Error Mean = mittlere Standardabweichung | | | | | | |

| (4) 2,5 mg Cilazapril plus 120 mg Propranolol | | | | | | |
|---|---|---|---|---|---|---|
| Proband/Std | -2 | 1 | 2 | 6 | 8 | 24 |
| 1 | 105 | 96 | 82 | 98 | 102 | 106 |
| 2 | 115 | 112 | 108 | 108 | 112 | 122 |
| 3 | 100 | 94 | 97 | 88 | 100 | 100 |
| 4 | 118 | 110 | 103 | 102 | 112 | 132 |
| 5 | 116 | 110 | 109 | 90 | 80 | 114 |
| 6 | 114 | 124 | 102 | 108 | 102 | 122 |
| Mittelwert | 1̅1̅1̅ | 1̅0̅8̅ | 1̅0̅0̅ | 9̅9̅ | 1̅0̅1̅ | 1̅1̅6̅ |
| SEM * | 3 | 5 | 4 | 4 | 5 | 5 |

| | | | | | | |
|---|---|---|---|---|---|---|
| * SEM = Standard Error Mean = mittlere Standardabweichung | | | | | | |

### Diastolische Blutdruckwerte in mmHg am 7. Versuchstag

| (1) Placebo plus Placebo | | | | | | |
|---|---|---|---|---|---|---|
| Proband/Std | -2 | 1 | 2 | 6 | 8 | 24 |
| 1 | 65 | 71 | 66 | 67 | 72 | 82 |
| 2 | 71 | 67 | 74 | 63 | 64 | 68 |
| 3 | 73 | 62 | 78 | 63 | 72 | 72 |
| 4 | 75 | 76 | 72 | 76 | 79 | 72 |
| 5 | 80 | 86 | 80 | 83 | 68 | 68 |
| 6 | 77 | 64 | 71 | 68 | 74 | 70 |
| Mittelwert | 7̅4̅ | 7̅1̅ | 7̅4̅ | 7̅0̅ | 7̅2̅ | 7̅2̅ |
| SEM * | 2 | 4 | 2 | 3 | 2 | 2 |

| | | | | | | |
|---|---|---|---|---|---|---|
| * SEM = Standard Error Mean = mittlere Standardabweichung | | | | | | |

| (2) 2,5 mg Cilazapril plus Placebo | | | | | | |
|---|---|---|---|---|---|---|
| Proband/Std | -2 | 1 | 2 | 6 | 8 | 24 |
| 1 | 63 | 63 | 54 | 58 | 52 | 78 |
| 2 | 78 | 80 | 77 | 52 | 62 | 64 |
| 3 | 69 | 62 | 66 | 57 | 64 | 78 |
| 4 | 75 | 72 | 69 | 68 | 72 | 66 |
| 5 | 76 | 72 | 73 | 76 | 78 | 72 |
| 6 | 75 | 82 | 73 | 64 | 66 | 88 |
| Mittelwert | 7̅3̅ | 7̅2̅ | 6̅9̅ | 6̅3̅ | 6̅6̅ | 7̅4̅ |
| SEM * | 2 | 3 | 3 | 4 | 4 | 4 |

| | | | | | | |
|---|---|---|---|---|---|---|
| * SEM = Standard Error Mean = mittlere Standardabweichung | | | | | | |

| (3) 120 mg Propranolol plus Placebo | | | | | | |
|---|---|---|---|---|---|---|
| Proband/Std | -2 | 1 | 2 | 6 | 8 | 24 |
| 1 | 67 | 56 | 58 | 50 | 64 | 74 |
| 2 | 78 | 64 | 68 | 54 | 54 | 76 |
| 3 | 67 | 74 | 75 | 64 | 62 | 76 |
| 4 | 75 | 66 | 71 | 56 | 80 | 78 |
| 5 | 74 | 68 | 67 | 76 | 79 | 72 |
| 6 | 72 | 52 | 64 | 58 | 50 | 78 |
| Mittelwert | 7̅2̅ | 6̅3̅ | 6̅7̅ | 6̅0̅ | 6̅5̅ | 7̅6̅ |
| SEM * | 2 | 3 | 2 | 4 | 5 | 1 |

| | | | | | | |
|---|---|---|---|---|---|---|
| * SEM = Standard Error Mean = mittlere Standardabweichung | | | | | | |

| (4) 2,5 mg Cilazapril plus 120 mg Propranolol | | | | | | |
|---|---|---|---|---|---|---|
| Proband/Std | -2 | 1 | 2 | 6 | 8 | 24 |
| 1 | 69 | 52 | 52 | 50 | 54 | 72 |
| 2 | 70 | 70 | 68 | 52 | 54 | 72 |
| 3 | 65 | 66 | 65 | 51 | 50 | 60 |
| 4 | 72 | 58 | 65 | 62 | 66 | 72 |
| 5 | 82 | 81 | 80 | 58 | 49 | 72 |
| 6 | 71 | 64 | 68 | 55 | 59 | 76 |
| Mittelwert | 7̅2̅ | 6̅5̅ | 6̅6̅ | 5̅5̅ | 5̅5̅ | 7̅1̅ |
| SEM * | 2 | 4 | 4 | 2 | 3 | 2 |

| | | | | | | |
|---|---|---|---|---|---|---|
| * SEM = Standard Error Mean = mittlere Standard abweichung | | | | | | |

B) Zehn männliche und drei weibliche Patienten mit einer essentiellen Hypertonie im Alter von 43-62 Jahren und mit einem Gewicht von 64-106 kg nahmen an dieser Crossover-Studie teil, die folgende drei Behandlungsabschnitte von jeweils 3 Wochen umfasste, wobei die Abschnitte (1) und (2) je nach Gruppe vertauscht waren:
   (1) 2,5 mg Cilazapril
   (2) 120 mg Propranolol
   (3) 2,5 mg Cilazapril plus 120 mg Propranolol.

Mit Ausnahme von drei Patienten erhielt keiner während mindestens 2 Wochen vor Beginn einer zweiwöchigen Placebo-Behandlung eine blutdrucksenkende Medikation. Nach dieser Zeit lag der diastolische Blutdruck beim Sitzen zwischen 95 und 120 mmHg. Dann wurden die Patienten wahlweise in zwei Gruppen eingeteilt, wobei die eine Gruppe zuerst mit Cilazapril und die andere zuerst mit Propranolol behandelt wurde. Nach der dreiwöchigen Behandlungsdauer wurde die Medikation in den beiden Gruppen ausgetauscht. Während der Behandlungsabschnitte wurden alle Messungen 2 Stunden nach Verabreichung durchgeführt, wobei der Blutdruck jeweils im Sitzen gemessen wurde.

Die Monotherapie mit Cilazapril bewirkte eine mittlere Senkung des diastolischen Blutdrucks um 8 mmHg und diejenige mit Propranolol eine solche um 9 mmHg, während die Kombinationstherapie eine Blutdrucksenkung um 19 mmHg bewirkte. Auf die Monotherapie mit Cilazapril sprachen 4 und auf diejenige mit Propranolol 2 von 13 Patienten an, während auf die Kombinationstherapie 10 von 13 Patienten ansprachen. Als "Ansprechen" auf eine Therapie wurde in dieser Studie eine Senkung des diastolischen Blutdrucks auf unter 90 mmHg defineriert.

Diese Ergebnisse zeigen, dargestellt am Beispiel der Kombination von Cilazapril mit Propranolol, die unerwartet vorteilhaften Eigenschaften der erfindungsgemässen Kombination. Bei Kenntnis des Standes der Technik konnte nämlich nicht erwartet werden, dass die Kombination von β-Blockern mit Pyridazodiazepinen eine so optimale blutdrucksenkende Wirkung zeigt.

Die erfindungsgemässe Kombination wird im allgemeinen oral, z.B. in Form von Tabletten, Lacktabletten, Dragées, Hart- und Weichgelatinekapseln, Lösungen, Emulsionen oder Suspensionen verabreicht. Die Verabreichung kann aber auch rektal, z.B. in Form von Suppositorien, oder parenteral, z.B. in Form von Injektionslösungen, erfolgen.

Zur Herstellung von Tabletten, Lacktabletten, Dragées und Hartgelatinekapseln kann eine erfindungsgemässe Kombination mit pharmazeutisch inerten anorganischen oder organischen Excipientien verarbeitet werden. Als solche Excipientien kann man z.B. für Tabletten, Dragées und Hartgelatinekapseln Lactose, Maisstärke oder Derivate davon, Talk, Stearinsäure oder deren Salze verwenden.

Für Weichgelatinekapseln eignen sich als Excipientien z.B. vegetabile Oele, Wachse, Fette, halbfeste und flüssige Polyole; je nach Beschaffenheit des Wirkstoffes sind jedoch bei Weichgelatinekapseln überhaupt keine Excipientien erforderlich.

Zur Herstellung von Lösungen und Sirupen eignen sich als Excipientien z.B. Wasser, Polyole, Saccharose, Invertzucker, Glucose und dergleichen.

Für Injektionslösungen eignen sich als Excipientien z.B. Wasser, Alkohole, Polyole, Glycerin und vegetabile Oele.

Für Suppositorien eignen sich als Excipientien z.B. natürliche oder gehärtete Oele, Wachse, Fette, halbflüssige oder flüssige Polyole und dergleichen.

Die pharmazeutischen Zubereitungen können daneben noch Konservierungsmittel, Lösevermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Die nachfolgenden Beispiele erläutern die Erfindung.

### Beispiel 1

Herstellung von Hartgelatinekapseln folgender Zusammensetzung:

| | |
|---|---|
| Cilazapril-monohydrat | 2,61 mg *) |
| Propranolol-hydrochlorid | 136,89 mg **) |
| Milchzucker pulv. | 33,50 mg |
| Milchzucker krist. | 101,00 mg |
| Maisstärke weiss | 20,00 mg |
| Talk | 5,00 mg |
| Magnesiumstearat | 1,00 mg |
| Total | 3̅0̅0̅,̅0̅0̅ m̅g̅ |

| | |
|---|---|
| *) entspricht 2,5 mg Cilazapril wasserfrei | |
| **) entspricht 120 mg Propranolol (Base) | |

### Herstellungsprinzip

Der Wirkstoff Cilazapril wird mit Milchzucker pulv. intensiv gemischt. Diese Vormischung wird mit dem Wirkstoff Propranolol, Milchzucker krist., Maisstärke weiss, Talk und Magnesiumstearat gemischt. Die Pulvermischung wird in Kapseln Grösse 1 gefüllt.

### Beispiele 2

Herstellung von Tabletten folgender Zusammensetzung:

| | |
|---|---|
| Cilazapril-monohydrat | 2,61 mg *) |
| Propranolol-hydrochlorid | 136,89 mg **) |
| Milchzucker pulv. | 120,00 mg |
| Maisstärke weiss | 40,50 mg |
| Polyvinylpyrrolidon | 4,00 mg |
| Maissstärke weiss | 40,00 mg |
| Talk | 5,00 mg |
| Magnesiumstearat | 1,00 mg |
| Total | 3̅5̅0̅,̅0̅0̅ m̅g̅ |

| | |
|---|---|
| *) entspricht 2,5 mg Cilazapril wasserfrei | |
| **) entspricht 120 mg Propranolol (Base) | |

### Herstellungsprinzip

Die Wirkstoffe werden mit Milchzucker pulv. und Maisstärke weiss gemischt. Die Mischung wird mit einer wässrigen Lösung von Polyvinylpyrrolidon befeuchtet und geknetet; die resultierende Masse wird granuliert, getrocknet und gesiebt. Das Granulat wird mit Maisstärke weiss (2. Teil), Talk und Magnesiumstearat gemischt und zu Tabletten geeigneter Grösse verpresst.

### Beispiel 3

Herstellung von Hartgelatinekapseln folgender Zusammensetzung:

| | |
|---|---|
| Cilazapril-monohydrat | 2,61 mg *) |
| Bisoprolol-fumarat (2:1) | 5,89 mg **) |
| Milchzucker pulv. | 80,50 mg |
| Milchzucker krist. | 110,00 mg |
| Maisstärke weiss | 50,00 mg |
| Talk | 30,00 mg |
| Magnesiumstearat | 1,00 mg |
| Total | 2̅8̅0̅,̅0̅0̅ m̅g̅ |

| | |
|---|---|
| *) entspricht 2,5 mg Cilazapril wasserfrei | |
| **) entspricht 5,0 mg Bisoprolol (Base) | |

### Herstellungsprinzip

Die Wirkstoffe Cilazapril-monohydrat und Bisoprolol-fumarat (2:1) werden mit Milchzucker pulv. (1. Teil) gemischt und gesiebt. Diese Vormischung wird mit dem Milchzucker (2. Teil), Milchzucker krist., Maisstärke weiss, Talk und Magnesiumstearat gemischt. Die Pulvermischung wird in Kapseln Grösse 2 abgefüllt.

### Beispiel 4

Herstellung von Lacktabletten folgender Zusammensetzung:

| Tablette: | |
|---|---|
| Cilazapril-monohydrat | 2,61 mg *) |
| Bisoprolol-fumarat (2:1) | 2,95 mg **) |
| Milchzucker pulv. | 227,44 mg |
| Maisstärke weiss | 100,00 mg |
| Polyvinylpyrrolidon | 10,00 mg |
| Talk | 5,00 mg |
| Magnesiumstearat | 2,00 mg |
| Total/Tablette | 3̅5̅0̅,̅0̅0̅ m̅g̅ |

| | |
|---|---|
| *) entspricht 2,5 mg Cilazapril wasserfrei | |
| **) entspricht 2,5 mg Bisoprolol (Base) | |

| Lackschicht: | |
|---|---|
| Hydroxypropylmethylcellulose | 2,80 mg |
| Talk | 2,00 mg |
| Polyäthylenglykol 6000 | 0,80 mg |
| Titandioxid | 0,20 mg |
| Eisenoxid rot | 0,20 mg |
| Total/Lackschicht | 6̅,̅0̅0̅ m̅g̅ |
| Total/Lacktablette | 356,00 mg |

### Herstellungsprinzip

### Tabletten

Die Wirkstoffe werden mit Milchzucker pulv. und Maisstärke weiss (1. Teil) gemischt. Die Mischung wird mit einer wässrigen Lösung von Polyvinylpyrrolidon befeuchtet und geknetet; die resultierende Masse wird granuliert, getrocknet und gesiebt. Das Granulat wird mit Maisstärke weiss (2. Teil), Talk und Magnesiumstearat gemischt und zu Tabletten geeigneter Grösse verpresst.

### Lackierung der Tabletten

Hydroxypropylmethylcellulose und Polyäthylenglykol 6000 werden in demineralisiertem Wasser (1. Teil) gelöst. In die Lösung wird eine Suspension von Talk, Titandioxid und Eisenoxid rot in Wasser (2. Teil) eingerührt. Die Lacksuspension wird in einer Dragiertrommel auf die Kerne gesprüht. Die Lacktabletten werden anschliessend getrocknet.

## Patentansprüche (Patentansprüche für folgende(n) Vertragsstaat(en): AT, BE, CH, DE, FR, GB, IT, LI, LU, NL, SE)

1. Pharmazeutische Zubereitung, dadurch gekennzeichnet, dass sie einen β-Blocker der allgemeinen Formel 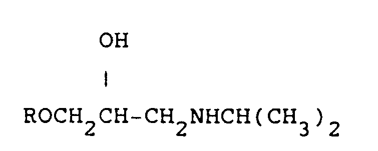 worin R Phenyl, welches gegebenenfalls durch nieder-Alkenyloxy, nieder-Alkoxy-nieder-alkyl, Aminocarbonyl-nieder-alkyl oder nieder-Alkoxy-nieder-alkoxy-nieder-alkyl substituiert ist, oder Naphthyl, Indolyl oder Dihydroxytetrahydronaphthyl bedeutet,
und ein Pyridazodiazepin der allgemeinen Formel 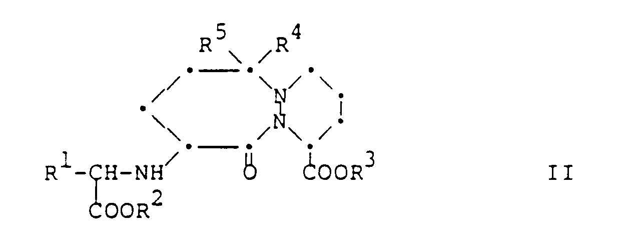 worin R¹ Aryl-nieder-alkyl, R² und R³ je Wasserstoff oder nieder-Alkyl und R⁴ und R⁵ je Wasserstoff oder zusammen eine Oxogruppe bedeuten, wobei die als "nieder" bezeichneten Reste 1-4 Kohlenstoffatome aufweisen,
in Form ihrer freien Basen, ihrer Hydrate oder ihrer pharmazeutisch verwendbaren Salze enthält.

2. Zubereitung nach Anspruch 1, dadurch gekennzeichnet, dass das Gewichtsverhältnis von β-Blocker zu Pyridazodiazepin 0,5:1 bis 500:1 in Bezug auf freie Base(n) beträgt.

3. Zubereitung nach Anspruch 2, dadurch gekennzeichnet, dass das Gewichtsverhältnis 1:1 bis 50:1 beträgt.

4. Zubereitung nach Anspruch 3, dadurch gekennzeichnet, dass das Gewichtsverhältnis 1:1 bis 5:1 beträgt.

5. Zubereitung nach einem der Ansprüche 1-4, dadurch gekennzeichnet, dass sie 5-320 mg eines β-Blockers und 1-5 mg eines Pyridazodiazepins oder äquivalente Mengen eines Hydrates oder eines pharmazeutisch verwendbaren Salzes als Tagesdosis enthält.

6. Zubereitung nach einem der Ansprüche 1-5, dadurch gekennzeichnet, dass sie einen β-Blocker der Formel I enthält, worin R α-Naphthyl, Indol-4-yl, 2-Allyloxyphenyl, 4-Methoxyäthylphenyl, 4-Aminocarbonylmethylphenyl, 4-Isopropyloxyäthoxymethylphenyl oder 6,7-Dihydroxy-5,6,7,8-tetrahydronaphth-1-yl bedeutet.

7. Zubereitung nach Anspruch 6, dadurch gekennzeichnet, dass sie Propranolol oder Bisoprolol als β-Blocker der Formel I enthält.

8. Zubereitung nach Anspruch 7, dadurch gekennzeichnet, dass sie Bisoprolol als β-Blocker der Formel I enthält.

9. Zubereitung nach einem der Ansprüche 1-8, dadurch gekennzeichnet, dass sie ein Pyridazodiazepin der Formel II enthält, worin R¹ Aryl-nieder-alkyl, R² nieder-Alkyl, R³ Wasserstoff und R⁴ und R⁵ je Wasserstoff oder zusammen eine Oxogruppe bedeuten.

10. Zubereitung nach Anspruch 9, dadurch gekennzeichnet, dass sie ein Pyridazodiazepin der Formel II enthält, worin R¹ Phenyl-nieder-alkyl, R² nieder-Alkyl und R³, R⁴ und R⁵ je Wasserstoff bedeuten.

11. Zubereitung nach Anspruch 10, dadurch gekennzeichnet, dass sie 9(S)-[1(S)-Aethoxycarbonyl-3-phenylpropylamino]octahydro -10-oxo-6H-pyridazo[1,2-a][1,2]diazepin-1(S)-carbonsäure als Pyridazodiazepin der Formel II enthält.

12. Zubereitung nach Anspruch 11, dadurch gekennzeichnet, dass die 9(S)-[1(S)-Aethoxycarbonyl-3-phenylpropylamino]octahydro -10-oxo-6H-pyridazo[1,2-a][1,2]diazepin-1(S)-carbonsäure als Salz oder Hydrat vorliegt.

13. Zubereitung nach Anspruch 12, dadurch gekennzeichnet, dass die 9(S)-[1(S)-Aethoxycarbonyl-3-phenylpropylamino]octahydro -10-oxo-6H-pyridazo[1,2-a][1,2]diazepin-1(S)-carbonsäure als Hydrobromid oder Monohydrat vorliegt.

14. Zubereitung nach einem der Ansprüche 1-13, dadurch gekennzeichnet, dass Propranolol als Hydrochlorid bzw. Bisoprolol als Fumarat vorliegt.

15. Verfahren zur Herstellung einer Zubereitung gemäss einem der Ansprüche 1-14, dadurch gekennzeichnet, dass man ein Gemisch der beiden Wirkstoffe in eine galenische Darreichungsform bringt.

16. Verwendung von einem β-Blocker der Formel I in Kombination mit einem ACE-Hemmer der Formel II zur Herstellung einer Zubereitung zur Behandlung der Hypertonie nach einem der Ansprüche 1-14.

## Patentansprüche (Patentansprüche für folgende(n) Vertragsstaat(en): ES, GR)

1. Verfahren zur Herstellung einer pharmazeutischen Zubereitung, dadurch gekennzeichnet, dass man ein Gemisch eines β-Blockers der allgemeinen Formel 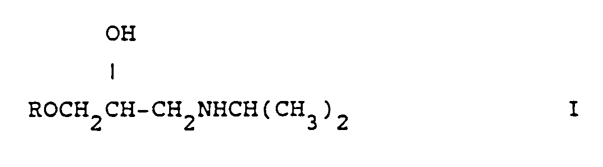 worin R Phenyl, welches gegebenenfalls durch nieder-Alkenyloxy, nieder-Alkoxy-nieder-alkyl, Aminocarbonyl-nieder-alkyl oder nieder-Alkoxy-nieder-alkoxy-nieder-alkyl substituiert ist, oder Naphthyl, Indolyl oder Dihydroxytetrahydronaphthyl bedeutet,
und eines Pyridazodiazepins der allgemeinen Formel 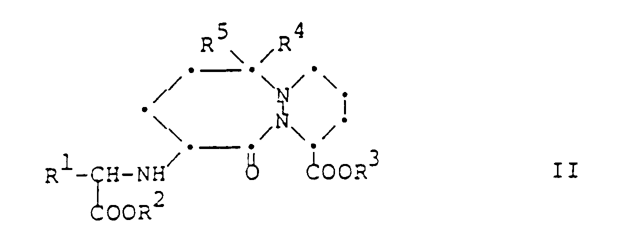 worin R¹ Aryl-nieder-alkyl, R² und R³ je Wasserstoff oder nieder-Alkyl und R⁴ und R⁵ je Wasserstoff oder zusammen eine Oxogruppe bedeuten, wobei die als "nieder" bezeichneten Reste 1-4 Kohlenstoffatome aufweisen,
in Form ihrer freien Basen, ihrer Hydrate oder ihrer pharmazeutisch verwendbaren Salze in eine galenische Darreichungsform bringt.

2. Verfahren nach Anspruch 1, dadurch gekennzeichnet, dass das Gewichtsverhältnis von β-Blocker zu Pyridazodiazepin 0,5:1 bis 500:1 in Bezug auf freie Base(n) beträgt.

3. Verfahren nach Anspruch 2, dadurch gekennzeichnet, dass das Gewichtsverhältnis 1:1 bis 50:1 beträgt.

4. Verfahren nach Anspruch 3, dadurch gekennzeichnet, das das Gewichtsverhältnis 1:1 bis 5:1 beträgt.

5. Verfahren nach einem der Ansprüche 1-4, dadurch gekennzeichnet, dass ein Gemisch von 5-320 mg eines β-Blockers und 1-5 mg eines Pyridazodiazepins oder äquivalente Mengen eines Hydrates oder eines pharmazeutisch verwendbaren Salzes zu einer Tagedosis verarbeitet wird.

6. Verfahren nach einem der Ansprüche 1-5, dadurch gekennzeichnet, dass ein β-Blocker der Formel I, worin R α-Naphthyl, Indol-4-yl, 2-Allyloxyphenyl, 4-Methyoxyäthylphenyl, 4-Aminocarbonylmethylphenyl, 4-Isopropyloxyäthoxymethylphenyl oder 6,7-Dihydroxy-5,6,7,8-tetrahydronaphth-1-yl bedeutet, verwendet wird.

7. Verfahren nach Anspruch 6, dadurch gekennzeichnet, dass Propranolol oder Bisoprolol als β-Blocker der Formel I verwendet wird.

8. Verfahren nach Anspruch 7, dadurch gekennzeichnet, dass Bisoprolol als β-Blocker der Formel I verwendet wird.

9. Verfahren nach einem der Ansprüche 1-8, dadurch gekennzeichnet, dass ein Pyridazodiazepin der Formel II, worin R¹ Aryl-nieder-alkyl, R² nieder-Alkyl, R³ Wasserstoff und R⁴ und R⁵ je Wasserstoff oder zusammen eine Oxogruppe bedeuten, verwendet wird.

10. Verfahren nach Anspruch 9, dadurch gekennzeich- net, dass ein Pyridazodiazepin der Formel II, worin R¹ Phenyl-nieder-alkyl, R² nieder-Alkyl und R³, R⁴ und R⁵ je Wasserstoff bedeuten, verwendet wird.

11. Verfahren nach Anspruch 10, dadurch gekennzeichnet, dass 9(S)-[1(S)-Aethoxycarbonyl-3-phenylpropylamino]octahydro -10-oxo-6H-pyridazo[1,2-a][1,2]diazepin-1(S)-carbonsäure als Pyridazodiazepin der Formel II verwendet wird.

12. Verfahren nach Anspruch 11, dadurch gekennzeichnet, dass die 9(S)-[1(S)-Aethoxycarbonyl-3-phenylpropylamino]octahydro -10-oxo-6H-pyridazo[1,2-a][1,2]diazepin-1(S)-carbonsäure als Salz oder Hydrat vorliegt.

13. Verfahren nach Anspruch 12, dadurch gekennzeichnet, dass die 9(S)-[1(S)-Aethoxycarbonyl-3-phenylpropylamino]octahydro -10-oxo-6H-pyridazo[1,2-a][1,2]diazepin-1(S)-carbonsäure als Hydrobromid oder Monohydrat vorliegt.

14. Verfahren nach einem der Ansprüche 1-13, dadurch gekennzeichnet, dass Propranolol als Hydrochlorid bzw. Bisoprolol als Fumarat vorliegt.

## Claims (Claims for the following Contracting State(s): AT, BE, CH, DE, FR, GB, IT, LI, NL, SE)

1. A pharmaceutical preparation, characterized that it contains a β-blocker of the general formula 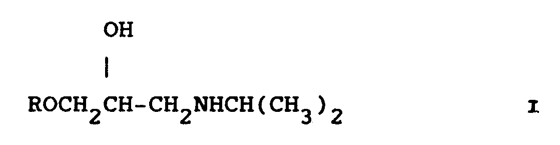 wherein R signifies phenyl, which is optionally substituted by lower-alkenyloxy, lower-alkoxy-lower-alkyl, aminocarbonyl-lower-alkyl or lower-alkoxy-lower-alkoxy-lower-alkyl, or naphthyl, indolyl or dihydroxytetrahydronaphthyl,
and a pyridazodiazepine of the general formula 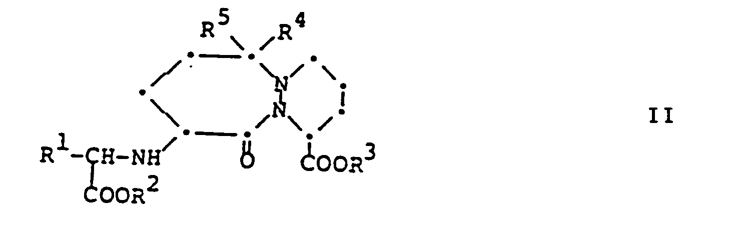 wherein R¹ signifies aryl-lower-alkyl, R² and R³ each signify hydrogen or lower-alkyl and R⁴ and R⁵ each signify hydrogen or together signify an oxo group, whereby the residues denoted as "lower" have 1-4 carbon atoms,
in the form of their free bases, their hydrates or their pharmaceutically usable salts.

2. A preparation according to claim 1, characterized in that the weight ratio of β-blocker to pyridazodiazepine amounts to 0.5:1 to 500:1 with respect to free base(s).

3. A preparation according to claim 2, characterized in that the weight ratio amounts to 1:1 to 50:1.

4. A preparation according to claim 3, characterized in that the weight ratio amounts to 1:1 to 5:1.

5. A preparation according to any one of claims 1-4, characterized in that it contains 5-320 mg of a β-blocker and 1-5 mg of a pyridazodiazepine or equivalent amounts of a hydrate or of a pharmaceutically usable salt as the daily dosage.

6. A preparation according to any one of claims 1-5, characterized in that it contains a β-blocker of formula I in which R signifies α-naphthyl, indol-4-yl, 2-allyloxyphenyl, 4-methoxyethylphenyl, 4-aminocarbonylmethylphenyl, 4-isopropyloxyethoxymethylphenyl or 6,7-dihydroxy-5,6.7,8-tetrahydronaphth-1-yl.

7. A preparation according to claim 6, characterized in that it contains propranolol or bisoprolol as the β-blocker of formula I.

8. A preparation according to claim 7, characterized in that it contains bisoprolol as the β-blocker of formula I.

9. A preparation according to any one of claims 1-8, characterized in that it contains a pyridazodiazepine of formula II in which R¹ signifies aryl-lower-alkyl, R signifies lower-alkyl, R³ signifies hydrogen and R⁴ and R⁵ each signify hydrogen or together signify an oxo group.

10. A preparation according to claim 9, characterized in that it contains a pyridazodiazepine of formula II in which R¹ signifies phenyl-lower-alkyl, R² signifies lower-alkyl and R³, R⁴ and R⁵ each signify hydrogen.

11. A preparation according to claim 10, characterized in that it contains 9(S)-[1(S)-ethoxycarbonyl-3-phenylpropylamino]octahydro-10-oxo-6H-pyridazo[1,2-a][1,2]diazepine-1(S)-carboxylic acid as the pyridazodiazepine of formula II.

12. A preparation according to claim 11, characterized in that the 9(S)-[1(S)-ethoxycarbonyl-3-phenylpropylamino]octahydro-10-oxo-6H-pyridazo[1,2-a][1,2]diazepine-1(S)-carboxylic acid is present as a salt or hydrate.

13. A preparation according to claim 12, characterized in that the 9(S)-[1(S)-ethoxycarbonyl-3-phenylpropylamino]octahydro-10-oxo-6H-pyridazo[1,2-a][1,2]diazepine-1(S)-carboxylic acid is present as the hydrobromide or monohydrate.

14. A preparation according to any one of claims 1-13, characterized in that propranolol is present as the hydrochloride and bisoprolol is present as the fumarate.

15. A process for the manufacture of a preparation in accordance with any one of claims 1-14, characterized by bringing a mixture of the two active substances into a galenical administration form.

16. The use of a β-blocker of formula I in combination with an ACE inhibitor of formula II for the manufacture of a preparation for the treatment of hypertension according to any one of claims 1-14.

## Claims (Claims for the following Contracting State(s): ES, GR)

1. A process for the manufacture of a pharmaceutical preparation, characterized by bringing a mixture of a β-blocker of the general formula 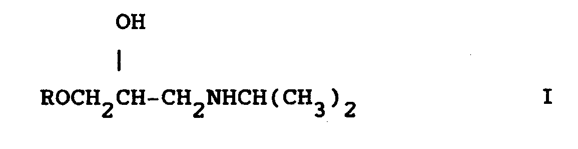 wherein R signifies phenyl, which is optionally substituted by lower-alkenyloxy, lower-alkoxy-lower-alkyl, aminocarbonyl-lower-alkyl or lower-alkoxy-lower-alkoxy-lower-alkyl, or naphthyl, indolyl or dihydroxytetrahydronaphthyl,
and a pyridazodiazepine of the general formula 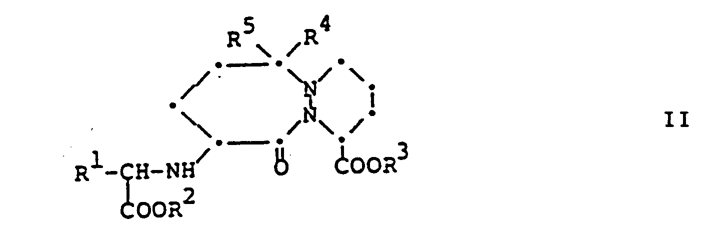 wherein R¹ signifies aryl-lower-alkyl, R² and R³ each signify hydrogen or lower-alkyl and R⁴ and R⁵ each signify hydrogen or together signify an oxo group, whereby the residues denoted as "lower" contain 1-4 carbon atoms,
in the form of their free bases, their hydrates or their pharmaceutically usable salts into a galenical administration form.

2. A process according to claim 1, characterized in that the weight ratio of β-blocker to pyridazodiazepine amounts to 0.5:1 to 500:1 with respect to free base(s).

3. A process according to claim 2, characterized in that the weight ratio amounts to 1:1 to 50:1.

4. A process according to claim 3, characterized in that the weight ratio amounts to 1:1 to 5:1.

5. A process according to any one of claims 1-4, characterized in that a mixture of 5-320 mg of a β-blocker and 1-5 mg of a pyridazodiazepine or equivalent amounts of a hydrate or of a pharmaceutically usable salt is processed to a daily dosage.

6. A process according to any one of claims 1-5, characterized in that a β-blocker of formula I in which R signifies α-naphthyl, indol-4-yl, 2-allyloxyphenyl, 4-methoxyethylphenyl, 4-aminocarbonylmethylphenyl, 4-isopropyloxyethoxymethylphenyl or 6,7-dihydroxy-5,6,7,8-tetrahydronaphth-1-yl is used.

7. A process according to claim 6, characterized in that propranolol or bisoprolol is used as the β-blocker of formula I.

8. A process according to claim 7, characterized in that bisoprolol is used as the β-blocker of formula I.

9. A process according to any one of claims 1-8, characterized in that a pyridazodiazepine of formula II in which R¹ signifies aryl-lower-alkyl, R² signifies lower-alkyl, R³ signifies hydrogen and R⁴ and R⁵ each signify hydrogen or together signify an oxo group is used.

10. A process according to claim 9, characterized in that a pyridazodiazepine of formula II in which R¹ signifies phenyl-lower-alkyl, R² signifies lower-alkyl and R³, R⁴ and R⁵ each signify hydrogen is used.

11. A process according to claim 10, characterized in that 9(S)-[1(S)-ethoxycarbonyl-3-phenylpropylamino]octahydro-10-oxo-6H-pyridazo[1,2-a][1,2]diazepine-1(S)-carboxylic acid is used as the pyridazodiazepine of formula II.

12. A process according to claim 11, characterized in that the 9(S)-[1(S)-ethoxycarbonyl-3-phenylpropylamino]octahydro-10-oxo-6H-pyridazo[1,2-a][1,2]diazepine-1(S)-carboxylic acid is present as a salt or hydrate.

13. A process according to claim 12, characterized in that the 9(S)-[1(S)-ethoxycarbonyl-3-phenylpropylamino]octahydro-10-oxo-6H-pyridazo[1,2-a][1,2]diazepine-1(S)-carboxylic acid is present as the hydrobromide or monohydrate.

14. A process according to any one of claims 1-13, characterized in that propranolol is present as the hydrochloride and bisoprolol is present as the fumarate.

## Revendications (Revendications pour l'(les) Etat(s) contractant(s) suivant(s): AT, BE, CH, DE, FR, GB, IT, LI, LU, NL, SE)

1. Préparation pharmaceutique, caractérisée en ce qu'elle contient un β-bloquant de formule générale : 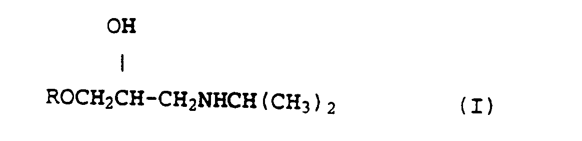 où R représente le phényle éventuellement substitué par un alcényle inférieur oxy, un alcoxy inférieur alkyle inférieur, un aminocarbonylalkyle inférieur ou un alcoxy inférieur alcoxy inférieur alkyle inférieur ou représente le naphtyle, l'indolyle ou le dihydroxytétrahydronaphtyle, et une pyridazodiazépine de formule générale : 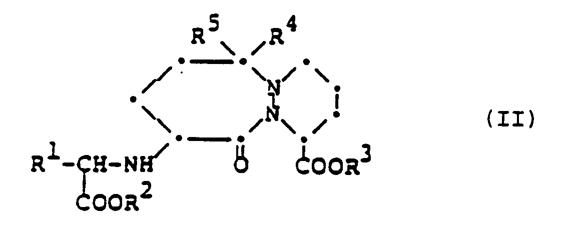 où R¹ représente un arylalkyle inférieur, R² et R³ représentent l'hydrogène ou un alkyle inférieur et R⁴ et R⁵ représentent l'hydrogène ou ensemble un groupe oxo, les restes désignés par "inférieurs" présentant 1 à 4 atomes de carbone,
sous forme de leurs bases libres, de leurs hydrates ou de leurs sels pharmaceutiquement utilisables.

2. Préparation selon la revendication 1, caractérisée en ce que le β-bloquant et la pyridazodiazépine sont dans un rapport pondéral compris entre 0,5:1 et 500:1 rapporté à la ou aux base(s) libre(s).

3. Préparation selon la revendication 2, caractérisée en ce que le rapport pondéral est compris entre 1:1 et 50:1.

4. Préparation selon la revendication 3, caractérisée en ce que le rapport pondéral est compris entre 1:1 et 5:1.

5. Préparation selon l'une des revendications 1 à 4, caractérisée en ce qu'elle contient 5 à 320 mg d'un β-bloquant et 1 à 5 mg d'une pyridazodiazépine ou d'une quantité équivalente d'un hydrate ou d'un sel pharmaceutiquement utilisable sous forme de dose journalière.

6. Préparation selon l'une des revendications 1 à 5, caractérisée en ce qu'elle contient un β-bloquant de formule I dans laquelle R représente l'α-naphtyle, l'indol-4-yle, le 2-allyloryphényle, le 4-méthoxyéthylphényle, le 4-aminocarbonylméthylphényle, le 4-isopropyloxyéthoxyméthylphényle ou le 6,7-dihydroxy-5,6,7,8-tétrahydronapht-1-yle.

7. Préparation selon la revendication 6, caractérisée en ce qu'elle contient du propranolol ou du bisoprolol comme β-bloquant de formule I.

8. Préparation selon la revendication 7, caractérisée en ce qu'elle contient du bisoprolol comme β-bloquant de formule I.

9. Préparation selon l'une des revendications 1 à 8, caractérisée en ce qu'elle contient une pyridazodiazépine de formule II dans laquelle R¹ représente un arylalkyle inférieur, R² un alkyle inférieur, R³ l'hydrogène et R⁴ et R⁵ l'hydrogène ou ensemble un groupe oxo.

10. Préparation selon la revendication 9, caractérisée en ce qu'elle contient une pyridazodiazépine de formule II dans laquelle R¹ représente un phénylalkyle inférieur, R² un alkyle inférieur et R³, R⁴ et R⁵ l'hydrogène.

11. Préparation selon la revendication 10, caractérisée en ce qu'elle contient l'acide 9(S)-[1(S)-éthoxycarbonyl-3-phénylpropylamino]-octahydro-10-oxo-6H-pyridazo[1,2-a][1,2]diazépine-1(S)-carboxylique comme pyridazodiazépine de formule II.

12. Préparation selon la revendication 11, caractérisée en ce qu'elle contient l'acide 9(S)-[1(S)-éthoxycarbonyl-3-phénylpropylamino]-octahydro-10-oxo-6H-pyridazo[1,2-a][1,2]diazépine-1(S)-carboxylique se présente sous forme de sel ou d'hydrate.

13. Préparation selon la revendication 12, caractérisée en ce qu'elle contient l'acide 9(S)-[1(S)-éthoxycarbonyl-3-phénylpropylamino]-octahydro-10-oxo-6H-pyridazo[1,2-a][1,2]diazépine-1(S)-carboxylique se présente sous forme de bromhydrate ou de monohydrate.

14. Préparation selon l'une des revendications 1 à 13, caractérisée en ce que le propanolol se présente sous forme de chlorhydrate et le bisoprolol sous forme de fumarate.

15. Procédé pour l'obtention d'une préparation selon l'une des revendications 1 à 14, caractérisé en ce que l'on met un mélange des deux substances actives sous la forme d'une présentation galénique.

16. Utilisation d'un β-bloquant de formule I en combinaison avec un inhibiteur d'ACE de formule II pour l'obtention d'une préparation pour le traitement de l'hypertonie selon l'une des revendications 1 à 14.

## Revendications (Revendications pour l'(les) Etat(s) contractant(s) suivant(s): ES, GR)

1. Procédé pour l'obtention d'une préparation pharmaceutique, caractérisé en ce que l'on met sous la forme d'une présentation galénique un mélange d'un β-bloquant de formule générale : 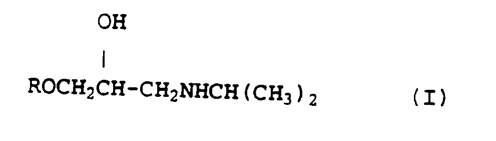 où R représente le phényle éventuellement substitué par un alcényle inférieur oxy, un alcoxy inférieur alkyle inférieur, un aminocarbonylalkyle inférieur ou un alcoxy inférieur alcoxy inférieur alkyle inférieur ou représente le naphtyle, l'indolyle ou le dihydroxytétrahydronaphtyle, et d'une pyridazodiazépine de formule générale : 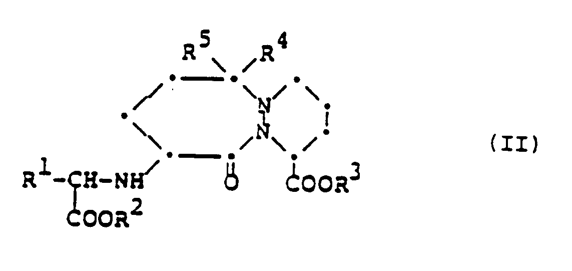 où R¹ représente un arylalkyle inférieur, R² et R³ représentent l'hydrogène ou un alkyle inférieur et R⁴ et R⁵ représentent l'hydrogène ou ensemble un groupe oxo, les restes désignés par "inférieurs" présentant 1 à 4 atomes de carbone,
sous forme de leurs bases libres, de leurs hydrates ou de leurs sels pharmaceutiquement utilisables.

2. Procédé selon la revendication 1, caractérisé en ce que le β-bloquant et la pyridazodiazépine sont dans un rapport pondéral compris entre 0,5:1 et 500:1 rapporté à la ou aux base(s) libre(s).

3. Procédé selon la revendication 2, caractérisé en ce que le rapport pondéral est compris entre 1:1 et 50:1.

4. Procédé selon la revendication 3, caractérisé en ce que le rapport pondéral est compris entre 1:1 et 5:1.

5. Procédé selon l'une des revendications 1 à 4, caractérisé en ce que l'on met sous forme de dose journalière un mélange de 5 à 320 mg d'un β-bloquant et de 1 à 5 mg d'une pyridazodiazépine ou d'une quantité équivalente d'un hydrate ou d'un sel pharmaceutiquement utilisable.

6. Procédé selon l'une des revendications 1 à 5, caractérisé en ce que l'on utilise un β-bloquant de formule I dans laquelle R représente l'α-naphtyle, l'indol-4-yle, le 2-allyloxyphényle, le 4-méthoxyéthylphényle, le 4-aminocarbonylméthylphényle, le 4-isopropyloxyéthoxyméthylphényle ou le 6,7-dihydroxy-5,6,7,8-tétrahydronapht-1-yle.

7. Procédé selon la revendication 6, caractérisé en ce que l'on utilise du propranolol ou du bisoprolol comme β-bloquant de formule I.

8. Procédé selon la revendication 7, caractérisé en ce que l'on utilise le bisoprolol comme β-bloquant de formule I.

9. Procédé selon l'une des revendications 1 à 8, caractérisé en ce que l'on utilise une pyridazodiazépine de formule II dans laquelle R¹ représente un arylalkyle inférieur, R² un alkyle inférieur, R³ l'hydrogène et R⁴ et R⁵ l'hydrogène ou ensemble un groupe oxo.

10. Procédé selon la revendication 9, caractérisé en ce que l'on utilise une pyridazodiazépine de formule II dans laquelle R¹ représente un phénylalkyle inférieur, R² un alkyle inférieur et R³, R⁴ et R⁵ l'hydrogène.

11. Procédé selon la revendication 10, caractérisé en ce que l'on utilise l'acide 9(S)-[1(S)-éthoxycarbonyl-3-phénylpropylamino]-octahydro-10-oxo-6H-pyridazo[1,2-a][1,2]diazépine-1(S)-carboxylique comme pyridazodiazépine de formule II.

12. Procédé selon la revendication 11, caractérisé en ce que l'on utilise l'acide 9(S)-[1(S)-éthoxycarbonyl-3-phénylpropylamino]-octahydro-10-oxo-6H-pyridazo[1,2-a][1,2]diazépine-1(S)-carboxylique sous forme de sel ou d'hydrate.

13. Procédé selon la revendication 12, caractérisé en ce que l'on utilise l'acide 9(S)-[1(S)-éthoxycarbonyl-3-phénylpropylamino]-octahydro-10-oxo-6H-pyridazo[1,2-a][1,2]diazépine-1(S)-carboxylique sous forme de bromhydrate ou de monohydrate.

14. Procédé selon l'une des revendications 1 à 13, caractérisé en ce que l'on utilise le propranolol sous forme de chlorhydrate et le bisoprolol sous forme de fumarate.
